# EUROPÄISCHE PATENTANMELDUNG

(11) **EP 0 745 898 A1**
(43) Veröffentlichungstag der Anmeldung: **04.12.1996**
(21) Anmeldenummer: 96107972.0
(22) Anmeldetag: 20.05.1996
(51) Int. Cl.: G03C 7/392, G03C 7/407

(54) **Verfahren zur Herstellung eines chromogen entwickelten farbfotografischen Bildes unter Verwendung einer Verbindung die mit primären aromatischen Aminen zu reagieren vermag**

(30) Priorität: 30.05.1995 DE 19519709
(71) Anmelder: Agfa-Gevaert AG, D-51373 Leverkusen (DE)
(72) Erfinder: Hübsch, Thomas, Dr., 51375 Leverkusen (DE); Schmuck, Arno, Dr., 42799 Leichlingen (DE)

(57) **Zusammenfassung**

Die Lagerstabilität fotografischer Farbbilder, die durch chromogene Entwicklung erhalten worden sind, läßt sich durch Verbindungen der Formel I, die mit primären Aminen, insbeondere Farbentwicklerverbindungen, zu reagieren vermögen, (Reagenz für primäre Amine) verbessern. Die Verbindungen der Formel I können dem farbbfotografischen Aufzeichnungsmaterial oder einem der Farbentwicklung nachgeschalteten Behandlungsbad zugestzt werden. Verbindungen der Formel I eignen sich außerdem als Zusatz zu einem gebrauchten fotografischen Behandlungsbad oder dessen Überlauf, um darin den Gehalt an Farbentwicklerverbindungen zu reduzieren, bevor das gebrauchte fotografische Behandlungsbad oder der Überlauf der Entsorgung oder der Wiederaufbereitung zugeführt wird. 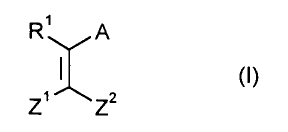 In Formel I bedeuten:
- A: -OR², -SR² oder -NR²R³;
- R¹: H, Alkyl, Aryl, Acyl, Sulfonyl oder einen Rest wie A oder einen Rest, der zusammen mit A oder zusammen mit Z¹ einen Ring vervollständigt;
- R²: H, Alkyl, Aryl, Acyl, Sulfonyl;
- R³: einen Rest wie R² oder gegebenenfalls einen Rest, der zusammen mit R² und dem N-Atom einen heterocyclischen Ring bildet,
- Z¹ und Z²: unabhängig voneinander: H, Halogen, Cyan, Alkyl, Aryl, Acyl, eine heterocyclische Gruppe, Alkoxycarbonyl, Aroxycarbonyl, Sulfonyl, Imino, Imido, Sulfamoyl, Carbonamido oder Sulfonamido; wobei Z¹ und Z² zusammen mit dem C-Atom, an das sie gebunden sind, ein carbocyclisches oder heterocyclisches Ringsystem bilden können.

## Beschreibung

Die Erfindung betrifft eine Verbindung, die mit primären, bevorzugt aromatischen Aminen zu reagieren vermag (Reagenz für primäre Amine). Die Erfindung betrifft weiterhin ein Verfahren zur Herstellung eines farbfotografischen Bildes durch chromogene Entwicklung unter Verwendung der erwähnten Verbindung, um die Lagerstabilität des erhaltenen Farbbildes zu verbessern. Schließlich betrifft die Erfindung ein Verfahren zur Verminderung des Gehaltes an Farbentwicklerverbindungen im Überlauf von Farbentwicklerbädern.

Die Lagerstabilität fotografischer Aufzeichnugsmaterialien vor und nach deren Verarbeitung ist ein wichtiges Qualitätsmerkmal. Gerade in tropischen Regionen mit hohen Temperaturen und/oder hoher Luftfeuchte kann sich ungenügende Stabilität in Erscheinungen wie Gradations- und Empfindlichkeitsverlust bei der Verarbeitung oder unerwünschter Dichtezunahme in verarbeitetem Material äußern. Ursache sind häufig Rückstände aus den zur Entwicklung fotografischer Materialien notwendigen Verarbeitungsbädern, die oft im Schichtverband verbleiben, besonders bei ungenügender Wässerung oder Verwendung eines Stabilisierbades. Gerade Reste von Farbentwicklerverbindungen des p-Phenylendiamin-Typs wirken sich hier störend aus, da diese bei Lagerung des verarbeiteten Materials durch Luftsauerstoff zu Entwickleroxidationsprodukten (EOP) oxidiert werden können und entweder durch chromogene Kupplung oder durch Zersetzungsreaktionen Vergilbung bewirken bzw. fotografischen Schleier erzeugen. Durch Zusatz von EOP-Fängern, beispielsweise vom Hydrochinon-Typ versucht man, die Lagerstabilität zu verbessern, wie unter anderem beschrieben in Research Disclosure, Dez. 1978, Nr. 17 643 und Dez. 1989, Nr. 308 119.

Bei Verwendung von 2-Äquivalentanilinopyrazolonkupplern in Colornegativ-Materialien ist ein bekanntes Problem die Zunahme der Magentadichte im verarbeiteten Material nach Lagerung unter tropischen Bedingungen, wie beispielsweise in WO 92/18903 beschrieben.

Ferner ist der Umgang mit fotografischen Abwässern auf Grund des Gehaltes an Farbentwicklerverbindungen problematisch, da p-Phenylendiamine allergene Reaktionen auslösen können und zudem giftig sind.

Die Verwendung von EOP-Fängern - hierbei handelt es sich meistens um nichtdiffundierene Hydrochinonverbindungen - zur Verbesserung der Lagerstabilität verarbeiteter fotografischer Materialen ist unvorteilhaft, da diese auch während des Entwicklungsprozesses EOP wieder reduzieren, was zur Folge hat, daß so die Oxidationskraft des Silberhalogenides nicht vollständig ausgenutzt wird. Weiter entstehen bei dieser Reaktion aus den EOP-Fängern Chinone, die selbst nicht lagerstabil sind und unter Verfärbung Zersetzungsreaktionen eingehen. Ebenso bleibt durch EOP-Fänger wieder reduzierter Entwickler im Schichtverband und kann dort erneut oxidiert werden. Die zur Vergilbung bzw. Schleiererhöhung führenden Prozesse laufen können dann erneut ablaufen.

Der Erfindung liegt die Aufgabe zugrunde, durch Zusatz spezieller Stabilisatoren die Lagerstabilität fotografischer Aufzeichnungsmaterialeien zu verbessern.Eine weitere Aufgabe besteht in der Bereitstellung eines Reagenzes zur irreversiblen Bindung von Aminen, insbeondere primären aromatischen Aminen und hier besonders Farbentwicklerverbindungen des p-Phenylendiamin-Typs.

Es wurde gefunden, daß Verbindungen der Formel I sich für die Lösung der genannten Aufgaben eignen. Insbesondere kann die Lagerstabilität verarbeiteter farbfotografischer Aufzeichnungsmaterialien durch Verwendung von Verbindungen der Formel I als Bildstabilisatoren wesentlich verbessert werden.

Gegenstand der Erfindung ist ein fotografisches Farbentwicklungsverfahren, bei dem ein farbfotografisches Aufzeichnungsmaterial mit mindestens einer bildmaßig belichteten Silberhalogenidemulsionsschicht in Gegenwart einer Farbkupplerverbindung und einer Farbentwicklerverbindung entwickelt wird, dadurch gekennzeichnet, daß das farbfotografische Aufzeichnungsmaterial und/oder ein dem Farbentwicklerbad nachgeschaltetes Behandlungsbad als Bildstabilisator eine Verbindung der folgenden Formel I enthält: 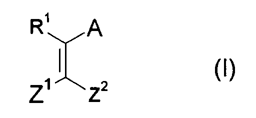 worin bedeuten:
- A: -OR², -SR² oder -NR²R³;
- R¹: H, Alkyl, Aryl, Acyl, Sulfonyl oder einen Rest wie A oder einen Rest, der zusammen mit A oder zusammen mit Z¹ einen Ring vervollständigt;
- R²: H, Alkyl, Aryl, Acyl, Sulfonyl;
- R³: einen Rest wie R² oder gegebenenfalls einen Rest, der zusammen mit R² und dem N-Atom einen heterocyclischen Ring bildet,
- Z¹ und Z²: unabhängig voneinander: H, Halogen, Cyan, Alkyl, Aryl, Acyl, eine heterocyclische Gruppe, Alkoxycarbonyl, Aroxycarbonyl, Sulfonyl, Imino, Imido, Sulfamoyl, Carbonamido oder Sulfonamido; wobei Z¹ und Z² zusammen mit dem C-Atom, an das sie gebunden sind, ein carbocyclisches oder heterocyclisches Ringsystem bilden können.

In einer bevorzugten Ausführungsform der Erfindung wird eine Verbindung einer der Formeln II und III als Bildstabilisator verwendet: 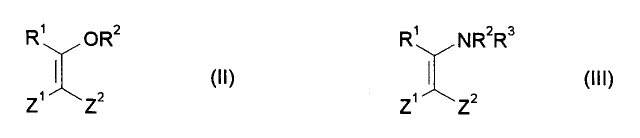 worin bedeuten:
- R¹: H, Alkyl, Aryl, -OR² oder -NR²R³;
- R²: Alkyl, Aryl oder (für -OR² auch): H;
- R³: einen Rest wie R² oder gegebenenfalls einen Rest, der zusammen mit R² und dem N-Atom einen heterocyclischen Ring bildet;
- Z¹ und Z²: unabhängig voneinander: H, Halogen, Cyan, Alkyl, Aryl, Acyl, eine heterocyclische Gruppe, Alkoxycarbonyl, Aroxycarbonyl, Sulfonyl, Imino, Imido, Sulfamoyl, Carbonamido oder Sulfonamido, wobei Z¹ und Z² zusammen mit dem C-Atom, an das sie gebunden sind, ein carbocyclisches oder heterocyclisches Ringsystem bilden können.

Ein durch R¹, R², Z¹ oder Z² dargestellter oder darin enthaltener Alkylrest ist geradkettig oder verzweigt und enthält 1 - 20 C-Atome; Beispiele sind Methyl, Ethyl, Propyl, Isopropyl, Butyl, t-Butyl, C₈-, C₁₀-, C₁₂-, C₁₃-, C₁₄-, C₁₅- und C₁₈-Alkyl. Ein solcher Alkylrest kann auch substituiert sein, z.B. mit Halogen wie Fluor oder Chlor, Phenyl (Beispiel: Benzyl), oder mit Alkoxycarbonyl.

Ein durch R¹, R², Z¹ oder Z² dargestellter oder darin enthaltener Arylrest ist beispielsweise Phenyl, gegebenenfalls substituiert, z.B. mit -NO₂, Alkyl oder Alkoxy.

Ein durch R¹, R², Z¹ oder Z² dargestellter Acylrest ist beispielsweise Alkylcarbonyl, Alkoxycarbonyl oder Arylcarbonyl.

Ein durch R¹, R², Z¹ oder Z² dargestellter Sulfonylrest ist beispielsweise Benzolsulfonyl.

Eine durch Z¹ oder Z² dargestellte heterocyclische Gruppe ist beispielsweise 4-Pyridyl. Ein durch Z¹, Z² und das C-Atom, an das sie gebunden sind, vervollstandigtes heterocyclisches Ringsystem ist beispielsweise Cyclohexandion, Indandion, Diazintrion oder Pyrazolon; solche Ringe können auch weiter substituiert sein, z.B. mit Alkyl, Aryl oder Acylamino.

Ein durch R¹ und A vervollständigter Ring ist beispielsweise ein Pyrimidin- oder Pyrrolidin-ring. Ein durch R¹ und Z¹ vervollständigter Ring ist beispielsweise ein Cyclohexenonring. Ein durch R² und R³ vervollständigter heterocyclischer Ring ist beispielsweise ein Piperidin- oder Indolring.

Beispiele für erfindungsgemäße Bildstabilisatoren der Formel I sind die folgenden:

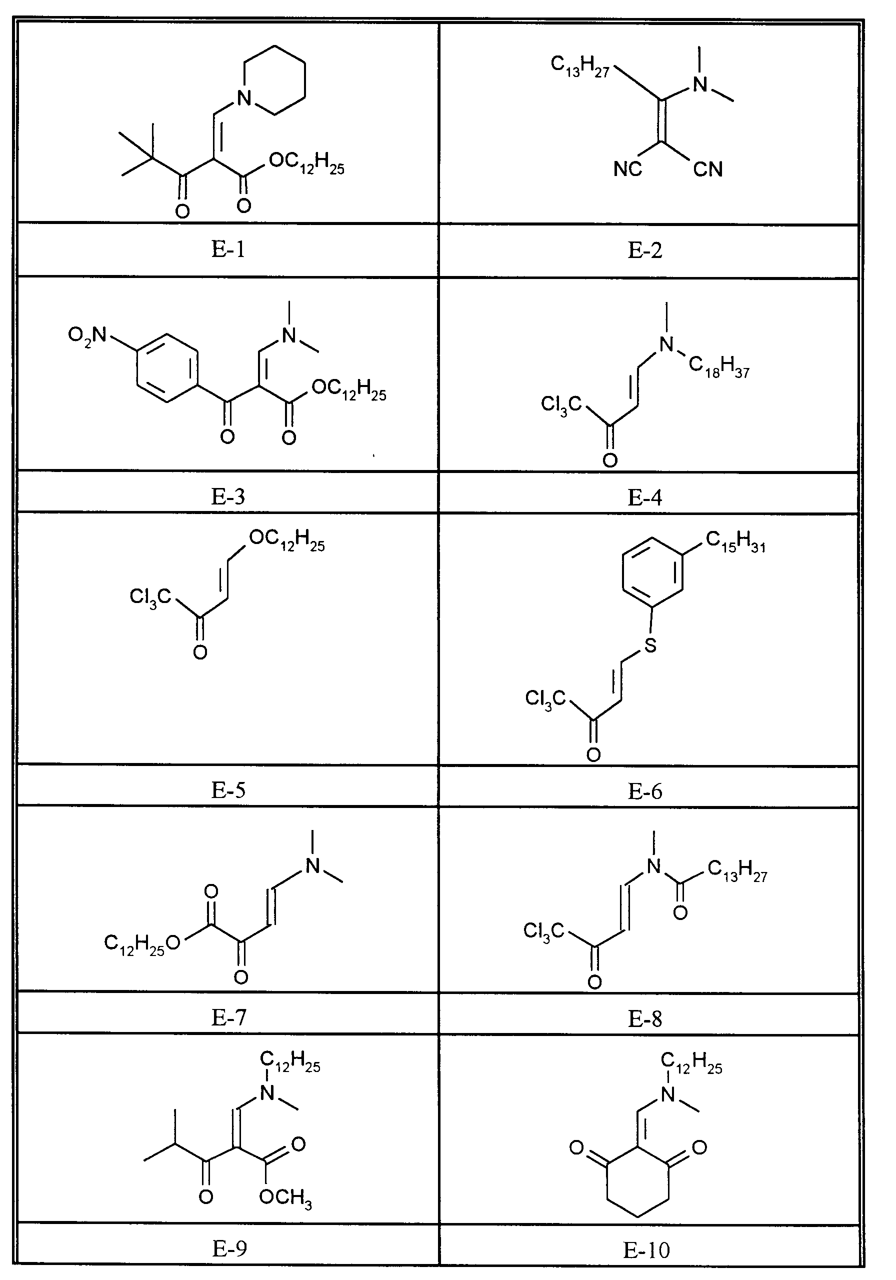

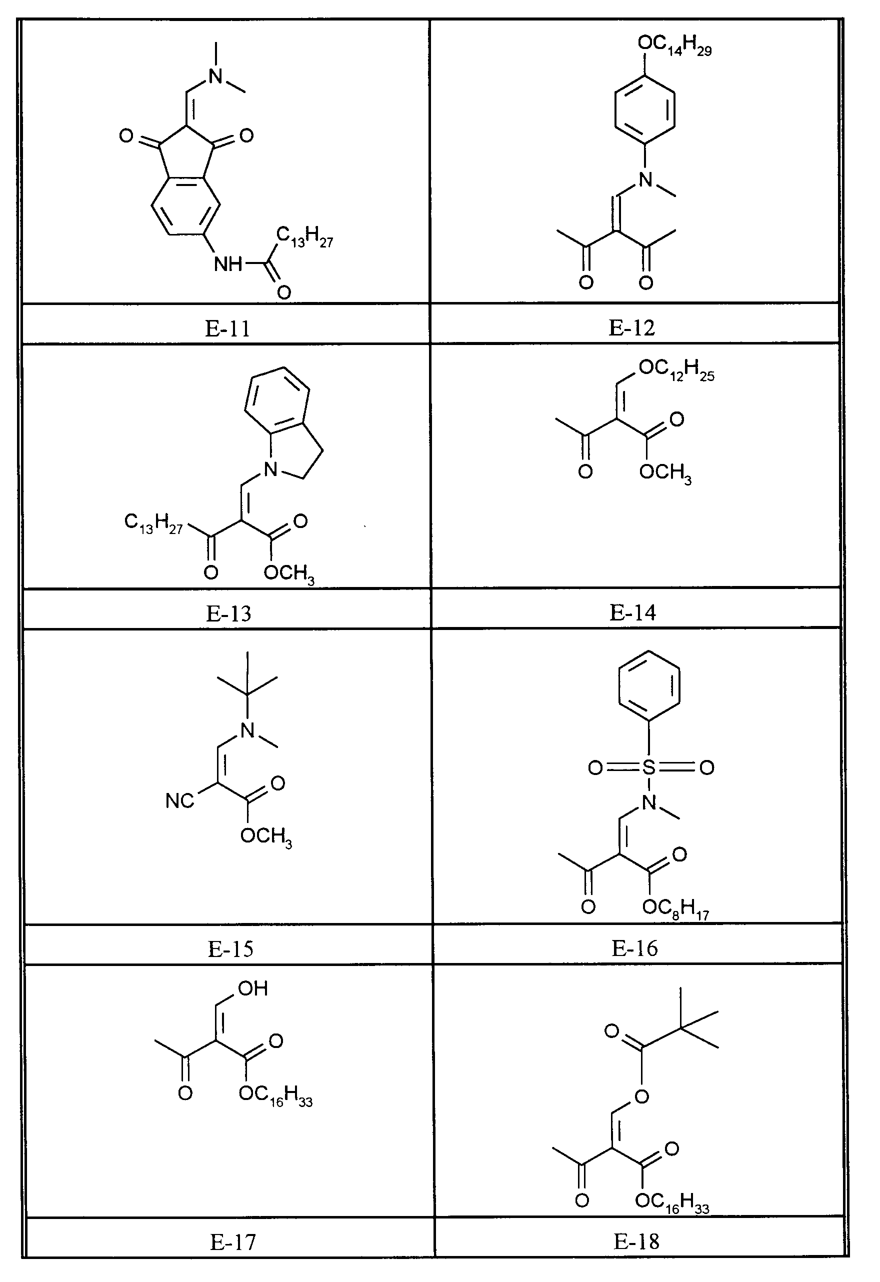

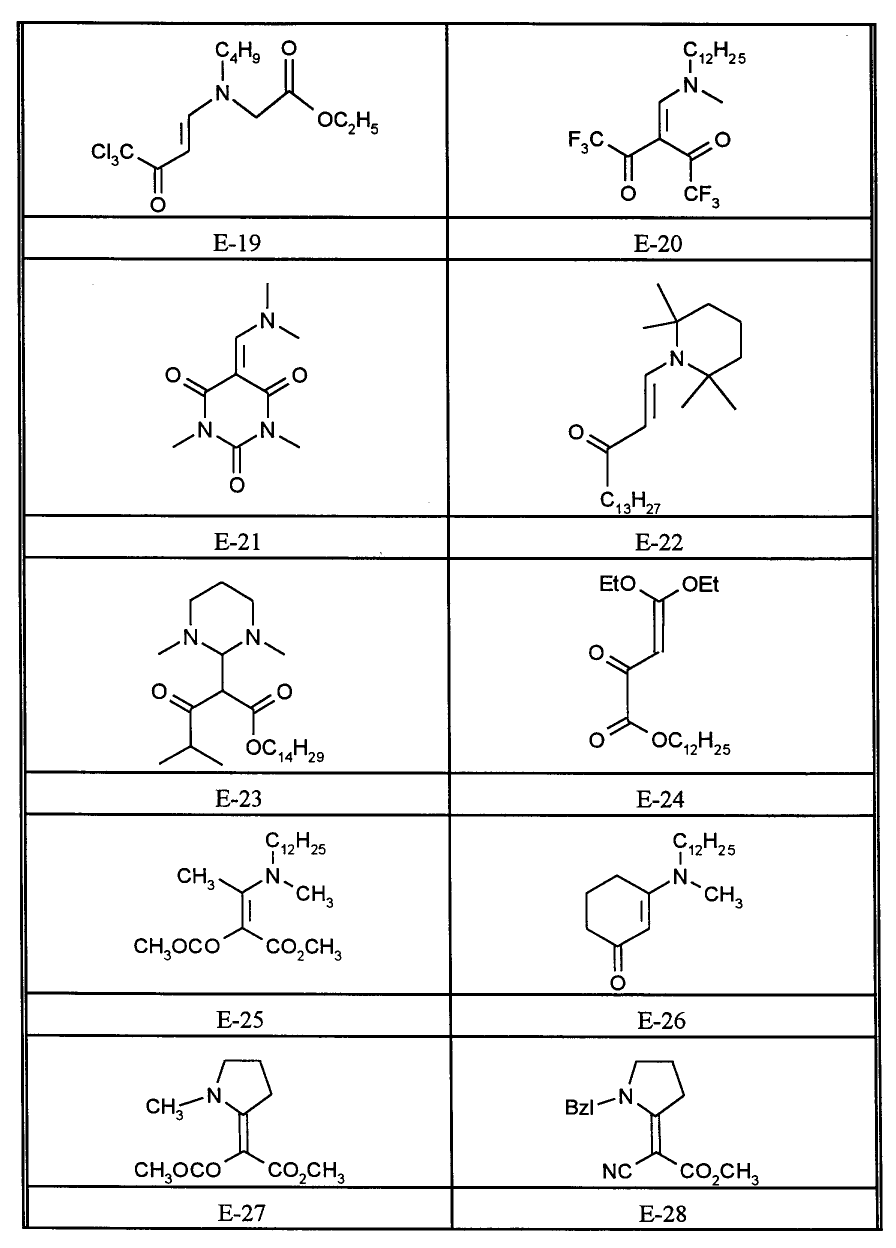

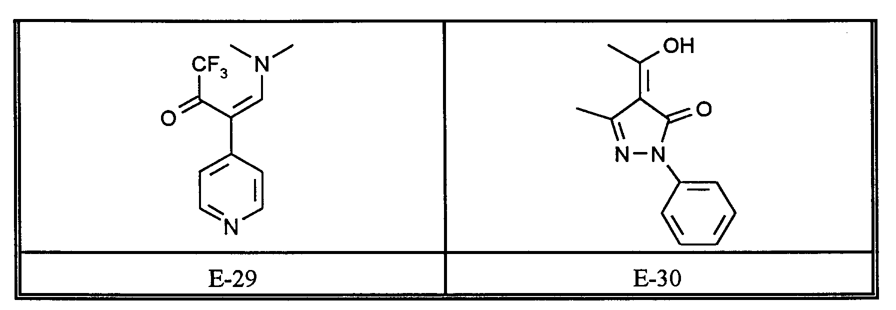

Bei den erfindungsgemäßen Verbindungen handelt es sich um konjugierte Systeme, für die zahlreiche kanonische Strukturen formuliert werden können. Einige davon sind an Hand von **E-10** beispielhaft dargestellt: 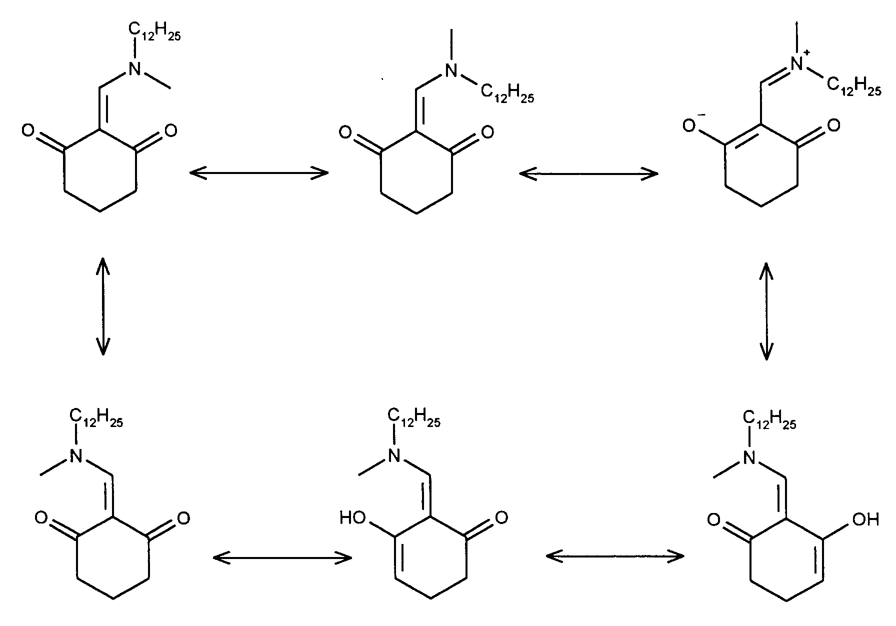 Herstellung von erfindungsgemäß zu verwendenden Verbindungen:
1. Synthese von **E-4**: 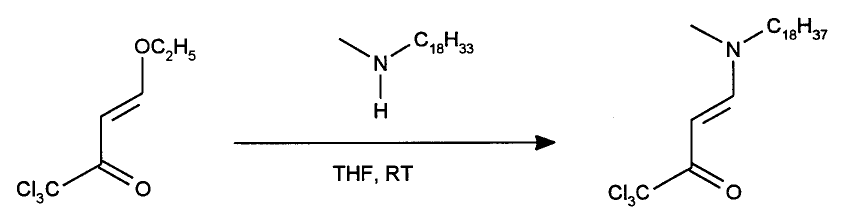 28,4 g (0,1 mol) N-Methylstearylamin (Aldrich) werden in 200 ml Ethanol gelöst. Man versetzt bei Raumtemperatur mit 22,8 g (0,105 mol) 1,1,1-Trichlor-4-ethoxy-but-3-en-4-on (Synthese siehe: Tietze et al., Org. Synth. (1990) S. 238 pp.). Die Temperatur steigt dabei auf ca. 35° C an und nach kurzer Zeit fällt ein kräftiger Niederschlag.
   Man kühlt auf 0° C, saugt ab, wäscht den Filterkuchen mit eiskaltem Ethanol nach und trocknet an der Luft.
   Ausbeute: 39,2 g **E-4**, entspechend 86 %.
2. Synthese von **E-14**: 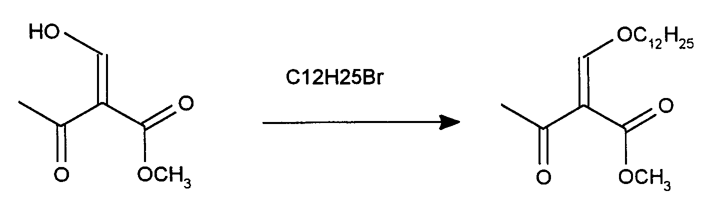 50 g (0,35 mol) 2-Formyl-acetessigsäuremethylester [CAS-Reg.-Nr. 131607-55-1] in 500 ml Dimethylformamid werden mit 59,5 g (0,35 mol) Silbernitrat versetzt, 30 min kräftig gerührt und der gelbliche Niederschlag abfiltriert.
   Dieser wird in 750 ml DMF unter kräftigem Rühren mit 87 g (0,35 mol) Dodecylbromid versetzt und über Nacht weitergerührt.
   Man filtriert, engt das Filtrat im Vakuum ein und arbeitet den Rückstand säulenchromatografisch auf.
   Ausbeute: 9,4 g, entsprechend 9 %.
3. Synthese von **E-28**:
   **E-28** kann nach Y. Shvo, H. Snanan-Afidi, *J*. *Am. Chem. Soc.* **91** (1969), 6683 pp., auf folgendem Weg synthetisiert werden: 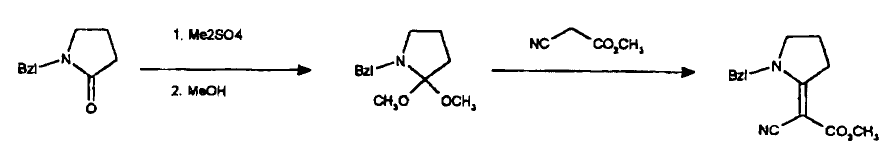
4. Synthese von **E-29**:
   **E-29** wird nach der Methode von Kuo et al., *J. Heterocyclic Chem.* **30** (1993) 37 pp. hergestellt: 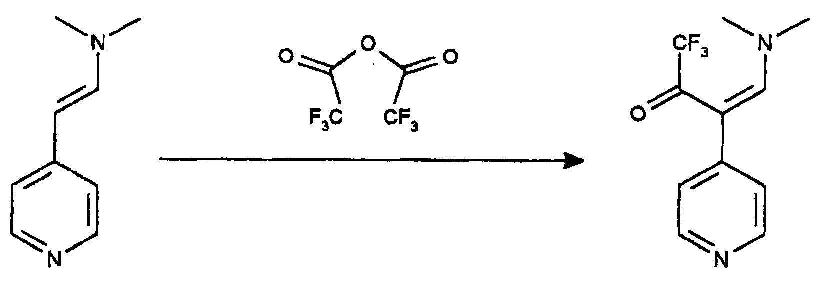
5. Synthese von **E-30**:
   **E-30** kann der Methode von Wolfbeis et al., *Z. Naturforsch.* **34b** (1979) 283 pp., folgend synthetisiert werden: 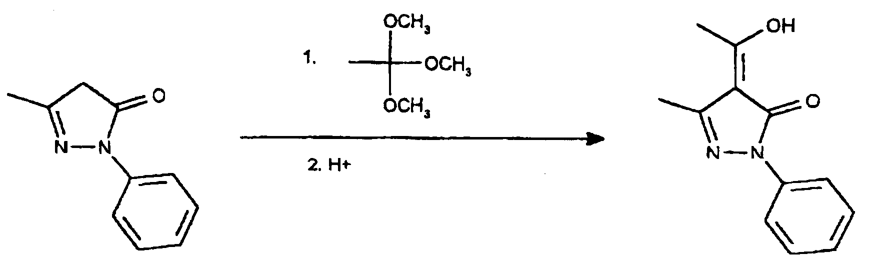 Dach Verwendung der Bildstabilisatoren gemäß vorliegender Erfindung wird sowohl die Schleierbildung in Reflektionsmaterialien (Vergilbung der Weißen) als auch die Erhöhung von Farbdichten bei Lagerung verarbeiteten Colormaterials zurückgedrängt. Die Verbindungen der Formel I können dabei sowohl dem farbfotografischen Aufzeichnungsmaterial, vorzugsweise einer oder mehreren der farbkupplerhaltigen Silberhalogenidemulsionsschichten oder auch einer oder mehreren nichtlichtempfindlichen Schichten, als auch einem der Farbentwicklung nachgeschalteten Verarbeitungsbad zugesetzt werden. Bei Zusatz zu dem farbfotografischen Aufzeichnungsmaterial beträgt die zugesetzte Menge vorzugsweise von 0,05 bis 5 g/m². Als der Farbentwicklung nachgeschaltetes Behandlungsbad kommt beispielsweise ein Bleichfixierbad oder Stabilisierbad in Frage; die zugesetzte Menge liegt in diesem Fall vorzugsweise zwischen 0,5 und 50 g/l.

In einem anderen Aspekt der vorliegenden Erfindung wird eine Verbindung der Formel I dem Überlauf eines gebrauchten fotografischen Behandlungsbades zugesetzt, bevor dieser gegebenenfalls nach einer Aufbewahrungszeit von bis zu einigen Stunden der Entsorgung oder der Wiederaufbereitung zugeführt wird. Bei diesen gebrauchten fotografischen Behandlungsbädern handelt es sich beispielsweise um ein Stopbad, ein Bleichbad, ein Fixierbad, ein Bleichfixierbad (blix), ein Stabilisierbad oder insbesondere ein Farbentwicklerbad.

Anstatt dem Überlauf kann die Verbindung der Formel I auch dem gebrauchten fotografischen Behandlungsbad selbst zugesetzt werden, wenn es sich um ein Behandlungsbad handelt, das dem Farbentwicklerbad nachgeschaltet ist.

Beispiele für farbfotografische Materialien sind Farbnegativfilme, Farbumkehrfilme, Farbpositivfilme, farbfotografisches Papier, farbumkehrfotografisches Papier, farbempfindliche Materialien für das Farbdiffusionstransfer-Verfahren oder das Silberfarbbleich-Verfahren.

Die fotografischen Materialien bestehen aus einem Träger, auf den wenigstens eine lichtempfindliche Silberhalogenidemulsionsschicht aufgebracht ist. Als Träger eignen sich insbesondere dünne Filme und Folien. Eine Übersicht über Trägermaterialien und auf deren Vorder- und Rückseite aufgetragene Hilfsschichten ist in Research Disclosure 37254, Teil 1(1995), S. 285 dargestellt.

Die farbfotografischen Materialien enthalten üblicherweise mindestens je eine rotempfindliche, grünempfindliche und blauempfindliche Silberhalogenidemulsionsschicht sowie gegebenenfalls Zwischenschichten und Schutzschichten.

Je nach Art des fotografischen Materials können diese Schichten unterschiedlich angeordnet sein. Dies sei für die wichtigsten Produkte dargestellt:

Farbfotografische Filme wie Colornegativfilme und Colorumkehrfilme weisen in der nachfolgend angegebenen Reihenfolge auf dem Täger 2 oder 3 rotempfindliche, blaugrünkuppelnde Silberhalogenidemulsionsschichten, 2 oder 3 grünempfindliche, purpurkuppelnde Silberhalogenidemulsionsschichten und 2 oder 3 blauempfindliche, gelbkuppelnde Silberhalogenidemulsionsschichten auf. Die Schichten gleicher spektraler Empfindlichkeit unterscheiden sich in ihrer fotografischen Empfindlichkeit, wobei die weniger empfindlichen Teilschichten in der Regel näher zum Träger angeordnet sind als die höher empfindlichen Teilschichten.

Zwischen den grünempfindlichen und blauempfindlichen Schichten ist üblicherweise eine Gelbfilterschicht angebracht, die blaues Licht daran hindert, in die darunter liegenden Schichten zu gelangen.

Die Möglichkeiten der unterschiedlichen Schichtanordnungen und ihre Auswirkungen auf die fotografischen Eigenschaften werden in J. Int. Rec. Mats., 1994, Vol. 22, Seiten 183-193 beschrieben.

Farbfotografisches Papier, das in der Regel wesentlich weniger lichtempfindlich ist als ein farbfotografischer Film, weist in der nachfolgend angegebenen Reihenfolge auf dem Träger üblicherweise je eine blauempfindliche, gelbkuppelnde Silberhalogenidemulsionsschicht, eine grünempfindliche, purpurkuppelnde Silberhalogenidemulsionsschicht und eine rotempfindliche, blaugrünkuppelnde Silberhalogenidemulsionsschicht auf; die Gelbfilterschicht kann entfallen.

Abweichungen von Zahl und Anordnung der lichtempfindlichen Schichten können zur Erzielung bestimmter Ergebnisse vorgenommen werden. Zum Beispiel können alle hochempfindlichen Schichten zu einem Schichtpaket und alle niedrigempfindlichen Schichten zu einem anderen Schichtpaket in einem fotografischen Film zusammengefaßt sein, um die Empfindlichkeit zu steigern (DE 25 30 645).

Wesentliche Bestandteile der fotografischen Emulsionsschichten sind Bindemittel, Silberhalogenidkörnchen und Farbkuppler.

Angaben über geeignete Bindemittel finden sich in Research Disclosure 37254, Teil 2 (1995), S. 286.

Angaben über geeignete Silberhalogenidemulsionen, ihre Herstellung, Reifüng, Stabilisierung und spektrale Sensibilisierung einschließlich geeigneter Spektralsensibilisatoren finden sich in Research Disclosure 37254, Teil 3 (1995), S. 286 und in Research Disclosure 37038, Teil XV (1995), S. 89.

Fotografische Materialien mit Kameraempfindlichkeit enthalten üblicherweise Silberbromidiodidemulsionen, die gegebenenfalls auch geringe Anteile Silberchlorid enthalten können. Fotografische Kopiermaterialien enthalten entweder Silberchloridbromidemulsionen mit bis 80 mol-% AgBr oder Silberchloridbromidemulsionen mit über 95 mol-% AgCl.

Angaben zu den Farbkupplern finden sich in Research Disclosure 37254, Teil 4 (1995), S. 288 und in Research Disclosure 37038, Teil II (1995), S. 80. Die maximale Absorption der aus den Kupplern und dem Farbentwickleroxidationsprodukt gebildeten Farbstoffe liegt vorzugsweise in den folgenden Bereichen: Gelbkuppler 430 bis 460 nm, Purpurkuppler 540 bis 560 nm, Blaugrünkuppler 630 bis 700 nm.

In farbfotografischen Filmen werden zur Verbesserung von Empfindlichkeit, Körnigkeit, Schärfe und Farbtrennung häufig Verbindungen eingesetzt, die bei der Reaktion mit dem Entwickleroxidationsprodukt Verbindungen freisetzen, die fotografisch wirksam sind, z.B. DIR-Kuppler, die einen Entwicklungsinhibitor abspalten.

Angaben zu solchen Verbindungen, insbesondere Kupplern, finden sich in Research Disclosure 37254, Teil 5 (1995), S. 290 und in Research Disclosure 37038, Teil XIV (1995), S. 86.

Die meist hydrophoben Farbkuppler, aber auch andere hydrophobe Bestandteile der Schichten, werden üblicherweise in hochsiedenden organischen Lösungsmitteln gelöst oder dispergiert. Diese Lösungen oder Dispersionen werden dann in einer wäßrigen Bindemittellösung (üblicherweise Gelatinelösung) emulgiert und liegen nach dem Trocknen der Schichten als feine Tröpfchen (0,05 bis 0,8 µm Durchmesser) in den Schichten vor.

Geeignete hochsiedende organische Lösungsmittel, Methoden zur Einbringung in die Schichten eines fotografischen Materials und weitere Methoden, chemische Verbindungen in fotografische Schichten einzubringen, finden sich in Research Disclosure 37254, Teil 6 (1995), S. 292.

Die in der Regel zwischen Schichten unterschiedlicher Spektralempfindlichkeit angeordneten nicht lichtempfindlichen Zwischenschichten können Mittel enthalten, die eine unerwünschte Diffusion von Entwickleroxidationsprodukten aus einer lichtempfindlichen in eine andere lichtempfindliche Schicht mit unterschiedlicher spektraler Sensibilisierung verhindern.

Geeignete Verbindungen (Weißkuppler, Scavenger oder EOP-Fänger) finden sich in Research Disclosure 37254, Teil 7 (1995), S. 292 und in Research Disclosure 37038, Teil III (1995), S. 84.

Das fotografische Material kann weiterhin UV-Licht absorbierende Verbindungen, Weißtöner, Abstandshalter, Filterfarbstoffe, Formalinfänger, Lichtschutzmittel, Antioxidantien, D_{Min}-Farbstoffe, Zusätze zur Verbesserung der Farbstoff-, Kuppler- und Weißenstabilität sowie zur Verringerung des Farbschleiers, Weichmacher (Latices), Biocide und anderes enthalten.

Geeignete Verbindungen finden sich in Research Disclosure 37254, Teil 8 (1995), S. 292 und in Research Disclosure 37038, Teile IV, V, VI, VII, X, XI und XIII (1995), S. 84 ff.

Die Schichten farbfotografischer Materialien werden üblicherweise gehärtet, d.h., das verwendete Bindemittel, vorzugsweise Gelatine, wird durch geeignete chemische Verfahren vernetzt.

Geeignete Härtersubstanzen finden sich in Research Disclosure 37254, Teil 9 (1995), S. 294 und in Research Disclosure 37038, Teil XII (1995), Seite 86.

Nach bildmäßiger Belichtung werden farbfotografische Materialien ihrem Charakter entsprechend nach unterschiedlichen Verfahren verarbeitet. Einzelheiten zu den Verfahrensweisen und dafür benötigte Chemikalien sind in Research Disclosure 37254, Teil 10 (1995), S. 294 sowie in Research Disclosure 37038, Teile XVI bis XXIII (1995), S. 95 ff. zusammen mit exemplarischen Materialien veröffentlicht.

### Beispiel 1

Ein mehrschichtiges farbfotografisches Aufzeichnungsmaterial wurde hergestellt, indem auf einen Schichtträger aus beidseitig mit Polyethylen beschichtetem Papier die folgenden Schichten in der angegebenen Reihenfolge aufgebracht wurden. Alle Mengenangaben beziehen sich auf 1 m², die Silbermenge ist als AgNO₃ angegeben:
- Schicht 1: (Substratschicht)
0,10 g Gelatine
- Schicht 2: (blauempfindliche Schicht):
blauempfindliche Silberhalogenidemulsion (99,5 mol-% Chlorid, 0,5 mol-% Bromid, mittlerer Korndurchmesser 0,9 µm) aus 0,50 g AgNO₃, mit
1,25 g Gelatine
0,42 g Gelbkuppler XY-1
0,18 g Gelbkuppler XY-2
0,50 g Trikresylphosphat (TKP)
0,10 g Stabilisator XST-1
0,70 mg Blausensibilisator XBS-1
0,30 mg Stabilisator XST-2
- Schicht 3: (Zwischenschicht)
1,10 g Gelatine
0,06 g Oxformfänger XSC-1
0,06 g Oxformfänger XSC-2
0,12 g TKP
- Schicht 4: (grünempfindliche Schicht)
grünempfindliche Silberhalogenidemulsion (99,5 mol-% Chlorid, 0,5 mol-% Bromid, mittlerer Korndurchmesser 0,47 µm) aus 0,40 g AgNO₃, mit
0,77 g Gelatine
0,21 g Magentakuppler XM-1
0,15 g Magentakuppler XM-2
0,05 g Magentakuppler XM-3
0,06 g Stabilisator XST-3
0,12 g Oxformfänger XSC-2
0,34 g Dibutylphthalat (DBP)
0,70 mg Grünsensibilisator XGS-2
0,50 mg Stabilisator XST-4
- Schicht 5: (UV-Schutzschicht)
1,15 g Gelatine
0,50 g UV-Absorber XUV-1
0,10 g UV-Absorber XUV-2
0,03 g Oxformfänger XSC-1
0,03 g Oxformfänger XSC-2
0,35 g TKP
- Schicht 6: (rotempfindliche Schicht)
rotempfindliche Silberhalogenidemulsion (99,5 mol-% Chlorid, 0,5 mol-% Bromid, mittlerer Korndurchmesser 0,5 µm) aus 0,30 g AgNO₃, mit
1,0 g Gelatine
0,40 g Cyankuppler XC-1
0,05 g Cyankuppler XC-2
0,46 g TKP
0,03 mg Rotsensibilisator XRS-3
0,60 mg Stabilisator XST-5
- Schicht 7: (UV-Schutzschicht)
0,35 g Gelatine
0,15 g UV-Absorber XUV-1
0,03 g UV-Absorber XUV-2
0,09 g TKP
- Schicht 8: (Schutzschicht)
0,90 g Gelatine
0,05 g Weißtöner XWT-1
0,07 g Beize (PVP)
1,20 mg Siliconöl
2,50 mg Abstandshalter (Polymethylmethacrylat, mittlere Teilchengröße 0,8 µm)
0,30 g Härtungsmittel XH-1

In Beispiel 1 verwendete Verbindungen:

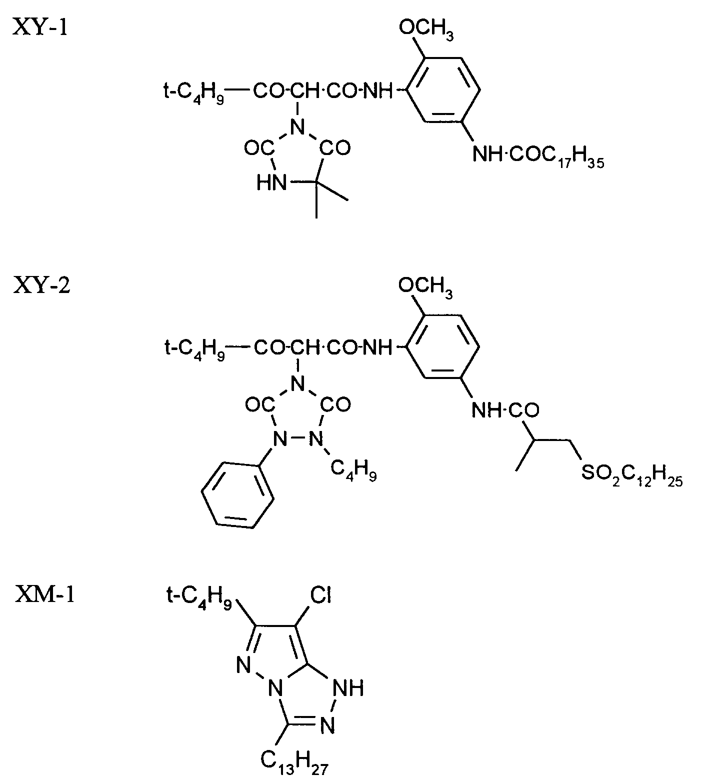

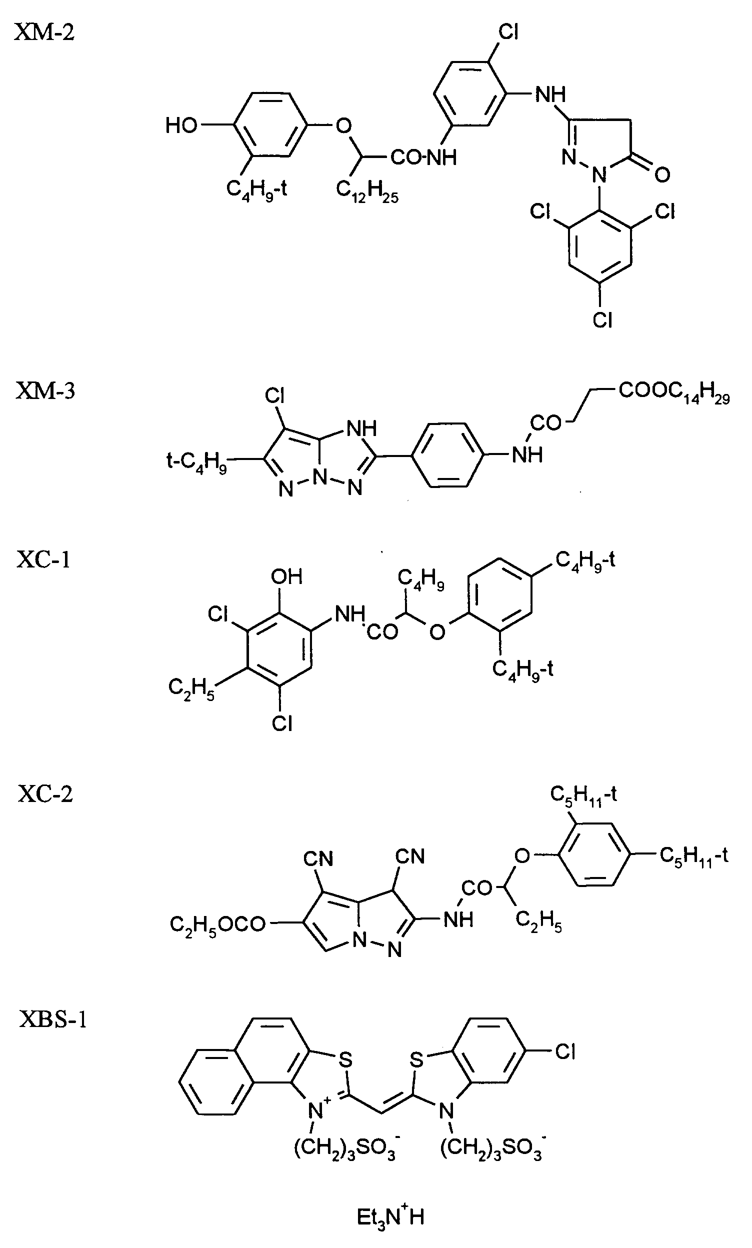

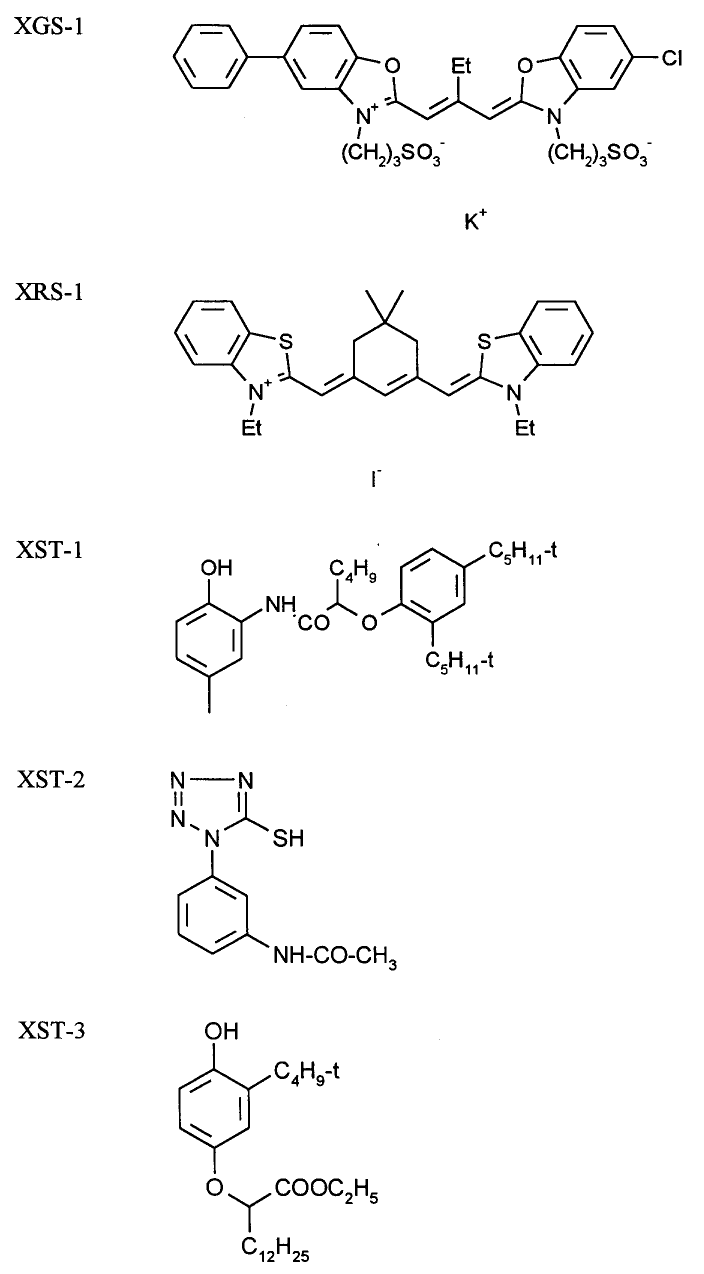

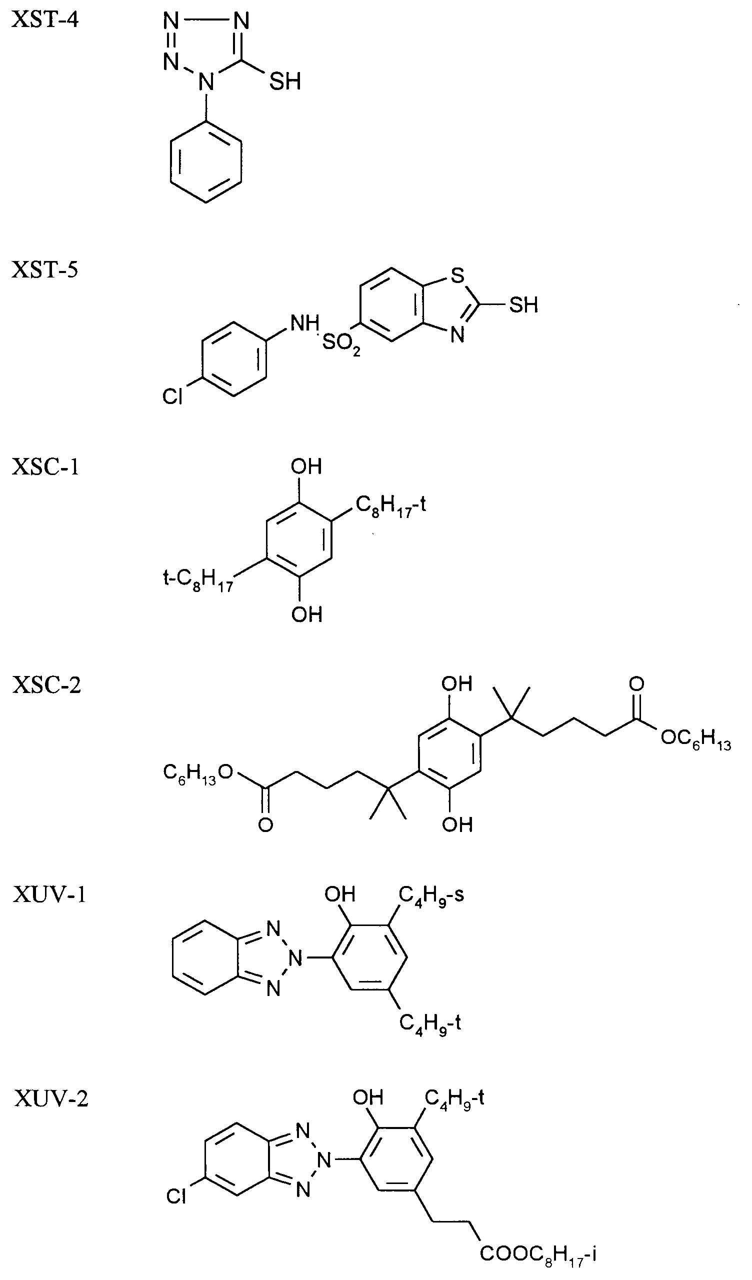

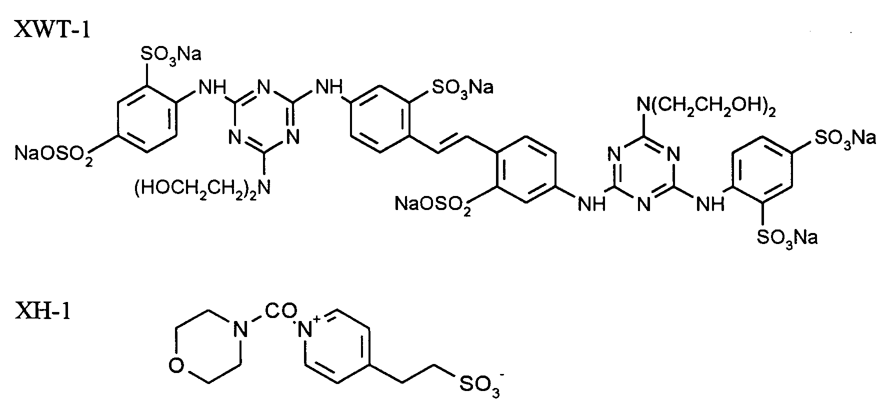

Das farbfotografische Aufzeichnungsmaterial wird durch einen Stufenkeil belichtet. Dabei werden zusätzliche Filter in den Strahlengang der Belichtungseinheit gebracht, so daß der Keil bei einer optischen Dichte von D = 0,6 neutral erscheint. Das belichtete Material wird nach folgendem Schema verarbeitet:

| Schritt | Zeit | Temperatur |
|---|---|---|
| Entwickeln | 45 s | 35° C |
| Bleichfixieren | 45 s | 35° C |
| Stabilisierbad | 90 s | 33° C |

Die Stabilisierung erfolgt in einem 4-Tank-Gegenstromverfahren. Die Verarbeitungsbäder wurden nach folgender Vorschrift angesetzt:

| Farbentwicklerlösung (CD) | |
|---|---|
| Tetraethylenglykol | 20,0 g |
| N,N-Diethylhydroxylamin | 4,0 g |
| (N-Ethyl-N-(2-methansulfonamido)ethyl))-4-amino-3-methylbenzolsulfat | 5,0 g |
| Kaliumsulfit | 0,2 g |
| Kaliumcarbonat | 30,0 g |
| Polymaleinsäureanhydrid | 2,5 g |
| Hydroxyethandiphosphonsäure | 0,2 g |
| Weißtöner (4,4'-Diaminostilbentyp) | 2,0 g |
| Kaliumbromid | 0,02 g |

auffüllen mit Wasser auf 1000 ml, pH-Wert mit KOH oder H₂SO₄ auf pH = 10,2 einstellen.

| Bleichfixierlösung (BX) | |
|---|---|
| Ammoniumthiosulfat | 75,0 g |
| Natriumhydrogensulfit | 13,5g |
| Ethylendiamintetraessigsäure (Fe-NH₄-Salz) | 45,0 g |

auffüllen mit Wasser auf 1000 ml, pH-Wert mit Ammoniak (25 %) oder Essigsäure auf pH 6,0 einstellen.

| Stabilisierbad (SB) | |
|---|---|
| Formalin (37%) | 0,1 g |
| Formalinsulfit-Addukt | 0,7 g |
| 5-Chlor-2-methyl-4-isothiazolin-3-on | 0,02 g |
| 2-Methyl-4-isothiazolin-3-on | 0,01 g |
| Kupfersulfat | 0,005 g |

auffüllen mit Wasser auf 1000 ml, pH-Wert auf 4,0 einstellen.

Es wurden Colorpapiermaterialien gemäß der oben genannten Rezeptur hergestellt, wobei in die in der Tabelle 1 genannten farbkupplerhaltigen Schichten jeweils E-4 zugesetzt wurde. Die belichteten und entwickelten Materialien wurden mit einem Auflichtdensitometer auf ihren fotografischen Schleier untersucht, vier Wochen bei 37° C gelagert und dann wurde der Schleieranstieg ΔDₘᵢₙ bestimmt.

**Tabelle 1**

| Nr. | Schicht, Menge E-4 | | Dₘᵢₙ | ΔDₘᵢₙ | |
|---|---|---|---|---|---|
| 1 | --- | gb | 121 | 23 | V |
| | | pp | 130 | 8 | |
| | | bg | 114 | 8 | |
| 2 | 6. Schicht, 130 mg/m² | gb | 121 | 17 | E |
| | | pp | 133 | 5 | |
| | | bg | 116 | 6 | |
| 3 | 6. Schicht, 65 mg/m² | gb | 122 | 18 | E |
| | | pp | 132 | 7 | |
| | | bg | 117 | 5 | |
| 4 | 4. Schicht, 70 mg/m² | gb | 123 | 12 | E |
| | | pp | 134 | 3 | |
| | | bg | 118 | 2 | |
| 5 | 4. Schicht, 35 mg/m² | gb | 122 | 16 | E |
| | | pp | 132 | 6 | |
| | | bg | 117 | 6 | |
| 6 | 2. Schicht, 80 mg/m² | gb | 121 | 15 | E |
| | | pp | 132 | 6 | |
| | | bg | 117 | 4 | |
| 7 | 2. Schicht, 40 mg/m² | gb | 122 | 15 | E |
| | | pp | 132 | 6 | |
| | | bg | 118 | 3 | |
| E = Erfindung, V = Vergleich gb = gelb, pp = magenta, bg = cyan | | | | | |

Durch den Einsatz von E-4 wird, unabhängig von der Schicht, in die es eingebracht wurde, eine signifikante Verminderung des Schleieranstiegs bewirkt.

### Beispiel 2

Das Material Nr. 1 aus Beispiel 1 wird über einen Stufenkeil belichtet und wie oben angegeben verarbeitet. Ein gleicher Keil wird analog verarbeitet, wobei das Bleichfixierbad (BX) zusätzlich 3,5 g bzw. das Stabilisierbad (SB) 1,8 g E-21 enthält. Beide Materialien werden mit einem Auflichtdensitometer auf ihren fotografischen Schleier untersucht, vier Wochen bei 37° C gelagert und dann wurde der Schleieranstieg ΔDₘᵢₙ bestimmt.

**Tabelle 2**

| Nr. | | | Dₘᵢₙ | ΔDₘᵢₙ | |
|---|---|---|---|---|---|
| 1 | Standardverarbeitung | gb | 122 | 25 | V |
| | | pp | 129 | 9 | |
| | | bg | 115 | 7 | |
| 2 | BX + 3,5 g/l E-21 | gb | 120 | 16 | E |
| | | pp | 128 | 6 | |
| | | bg | 113 | 6 | |
| 3 | SB + 1,9 g/l E-21 | gb | 123 | 15 | E |
| | | pp | 127 | 6 | |
| | | bg | 113 | 7 | |

Durch Zusatz von E-21 im BX- oder SB-Bad wird eine deutliche Verbesserung der Lagerstabilität des fotografischen Materials erzielt.

### Beispiel 3

0,06 mol Magentakuppler XM-4 wurden mit 0,05 mol XW- 1 und 35 g Ölbildner TKP wie in Tabelle 1 angegeben in 400 ml 10 %iger Gelatinelösung dispergiert und mit 1 kg einer rotsensibilisierten Silberbromidiodidemulsion (4 mol % Iodid, mittlerer Korndurchmesser 0,45 µm) aus 132 g AgNO₃ und 45 g Gelatine vermischt.

Das Gemisch wurde anschließend auf eine Cellulosetriacetatfolie vergossen mit einem Silberauftrag von 3,2 g (AgNO₃/m²). Nach Härtung mit einem Carbamoylpyridiniumsalz (XH-1) über eine zusätzliche Schutzschicht wurde das so hergestellte Material hinter einem graduierten Stufenkeil belichtet und wie in The British Journal of Photography, 1974, S. 597, beschrieben, verarbeitet.

Außer XM-4 wurden in gleicher Weise zwei weitere Magentakuppler XM-5 und XM-6 geprüft.

Die in Beispiel 3 verwendeten Verbindungen haben folgende Formeln:

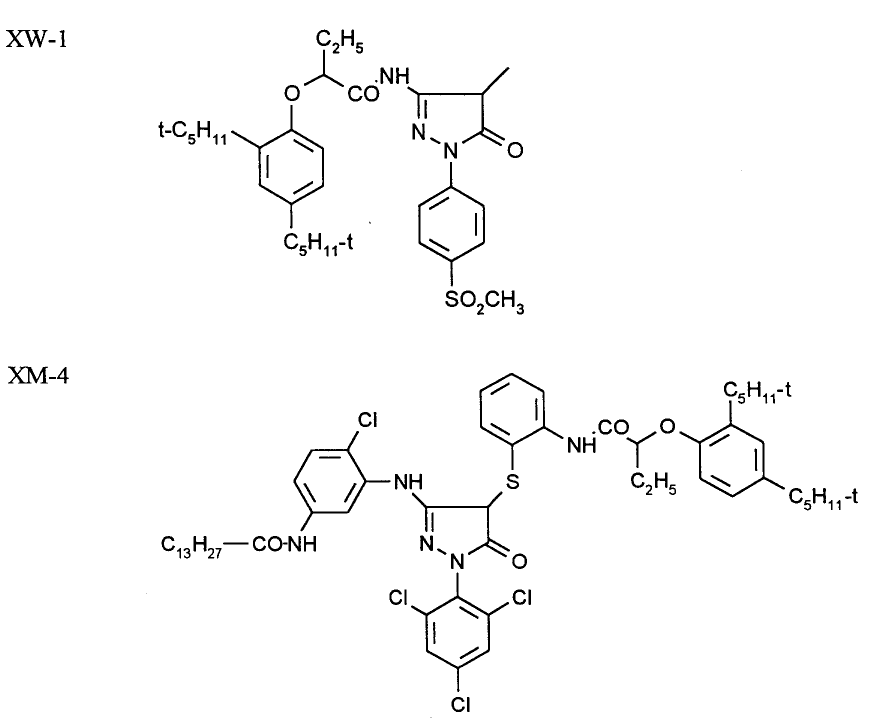

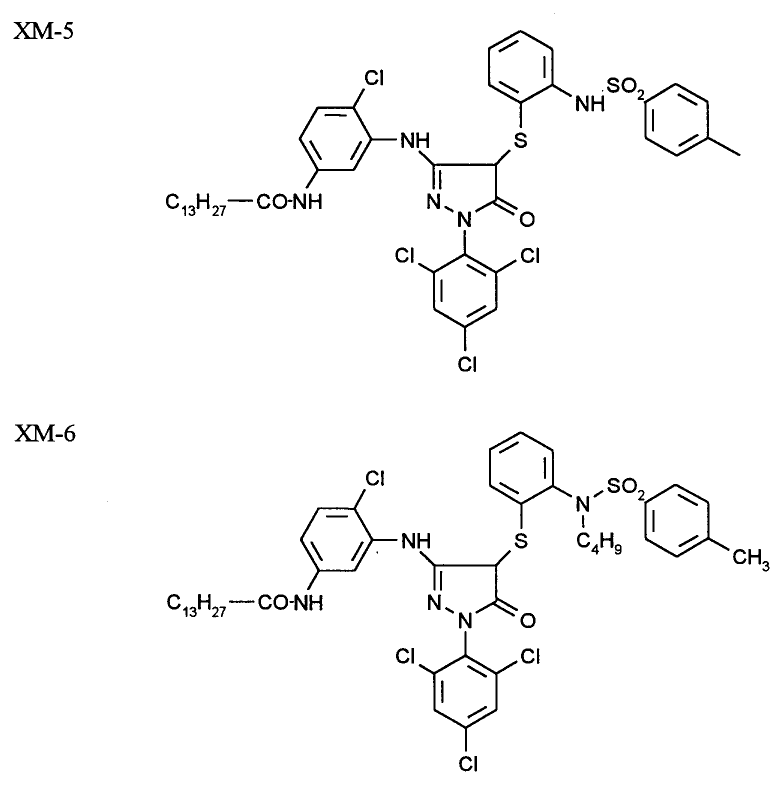

Nach Bestimmung der Frischsensitometrie wurde das verarbeitete Material bei 60°C, 95 % rel. Feuchte 5 Tage gelagert und anschließend wurde die Magentadichte im Schleier sowie in Dₘₐₓ erneut bestimmt. Der Unterschied ist als ΔDₘᵢₙ bzw. ΔDₘₐₓ in Tabelle 3 aufgeführt. Weiter wurden drei gleich aufgebaute Materialien hergestellt, die neben dem jeweiligen Magentakuppler noch 0,02 mol des erfindungsgemäßen Stabilisators E-4 enthalten, und ebenfalls der oben beschriebenen Lagerprüfung unterworfen (Tabelle 3).

**Tabelle 3**

| | Dₘᵢₙ | Dₘₐₓ | ΔDₘᵢₙ | ΔDₘₐₓ | |
|---|---|---|---|---|---|
| XM-4 | 27 | 199 | 28 | 25 | Vergleich |
| XM-5 | 20 | 244 | 31 | 54 | Vergleich |
| XM-6 | 18 | 219 | 24 | 23 | Vergleich |
| XM-4 + E-4 | 25 | 200 | 18 | 19 | Erfindung |
| XM-5 + E-4 | 21 | 229 | 22 | 32 | Erfindung |
| XM-6 + E-4 | 18 | 215 | 18 | 18 | Erfindung |

Man erkennt, daß durch Zusatz von E-4 deutliche Vorteile bezüglich Dichteanstieg bei Tropenlagerung nach Verarbeitung erzielt werden.

### Beispiel 4

In 1 l Entwicklerüberlauf aus dem C-41-Filmentwicklungsprozeß (The British Journal of Photography, 1974, S. 597) wurde der Gehalt an Farbentwickler CD-4 durch HPLC bestimmt (Tabelle 4, Eintrag 1).
Dann wurden unter starkem Rühren 11 g E-21 hinzugefügt, über Nacht stehen gelassen und der CD-4-Gehalt erneut gemessen (Eintrag 2).

**Tabelle 4**

| | Gehalt an CD-4 (g/l) | |
|---|---|---|
| 1 | 5,2 | Vergleich |
| 2 | < 0,2 | Erfindung |

Durch Verwendung der erfindungsgemäßen Verbindungen läßt sich der Entwicklergehalt fotografischer Bäder drastisch herabsetzen.

## Patentansprüche

1. Fotografisches Farbentwicklungsverfahren, bei dem ein farbfotografisches Aufzeichnungsmaterial mit mindestens einer bildmaßig belichteten Silberhalogenidemulsionsschicht in Gegenwart einer Farbkupplerverbindung und einer Farbentwicklerverbindung entwickelt wird, dadurch gekennzeichnet, daß das farbfotografische Aufzeichnungsmaterial und/oder ein dem Farbentwicklerbad nachgeschaltetes Behandlungsbad eine Verbindung der folgenden Formel enthält: 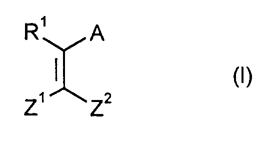 worin bedeuten
A -OR², -SR² oder -NR²R³;
R¹ H, Alkyl, Aryl, Acyl, Sulfonyl oder einen Rest wie A oder einen Rest, der zusammen mit A oder zusammen mit Z¹ einen Ring vervollständigt;
R² H, Alkyl, Aryl, Acyl, Sulfonyl;
R³ einen Rest wie R² oder gegebenenfalls einen Rest, der zusammen mit R² und dem N-Atom einen heterocyclischen Ring bildet,
Z¹ und Z² unabhängig voneinander: H, Halogen, Cyan, Alkyl, Aryl, Acyl, eine heterocyclische Gruppe, Alkoxycarbonyl, Aroxycarbonyl, Sulfonyl, Imino, Imido, Sulfamoyl, Carbonamido oder Sulfonamido; wobei Z¹ und Z² zusammen mit dem C-Atom, an das sie gebunden sind, ein carbocyclisches oder heterocyclisches Ringsystem bilden können.

2. Farbentwicklungsverfahren nach Anspruch 1, dadurch gekennzeichnet, daß das farbfotografische Aufzeichnungsmaterial und/oder ein dem Farbentwicklerbad nachgeschaltetes Behandlungsbad eine Verbindung einer der Formel II und III enthält 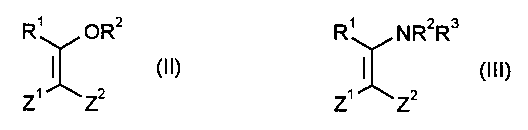 worin bedeuten:
R¹ H, Alkyl, Aryl, -OR² oder -NR²R³;
R² Alkyl, Aryl oder (für -OR² auch): H;
R³ einen Rest wie R² oder gegebenenfalls einen Rest, der zusammen mit R² und dem N-Atom einen heterocyclischen Ring bildet;
Z¹ und Z² unabhängig voneinander: H, Halogen, Cyan, Alkyl, Aryl, Acyl, eine heterocyclische Gruppe, Alkoxycarbonyl, Aroxycarbonyl, Sulfonyl, Imino, Imido, Sulfamoyl, Carbonamido oder Sulfonamido, wobei Z¹ und Z² zusammen mit dem C-Atom, an das sie gebunden sind, ein carbocyclisches oder heterocyclisches Ringsystem bilden können.

3. Farbfotografisches Aufzeichnungsmaterial mit einem Schichtträger und darauf angeordnet mindestens einer rotempfindlichen Silberhalogenidemulsionsschicht, der ein Cyankuppler zugeordnet ist, mindestens einer grünempfindlichen Silberhalogenidemulsionsschicht, der ein Magentakuppler zugeordnet ist, mindestens einer blauempfindlichen Silberhalogenidemulsionsschicht, der ein Gelbkuppler zugeordnet und gegebenenfalls weiteren Schichten, dadurch gekennzeichnet, daß es in mindestens einer seiner kupplerhaltigen Schichten eine Verbindung der Formel I enthält: 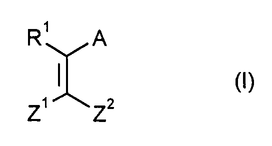 worin bedeuten
A -OR², -SR² oder -NR²R³;
R¹ H, Alkyl, Aryl, Acyl, Sulfonyl oder einen Rest wie A oder einen Rest, der zusammen mit A oder zusammen mit Z¹ einen Ring vervollständigt;
R² H, Alkyl, Aryl, Acyl, Sulfonyl;
R³ einen Rest wie R² oder gegebenenfalls einen Rest, der zusammen mit R² und dem N-Atom einen heterocyclischen Ring bildet,
Z¹ und Z² unabhängig voneinander: H, Halogen, Cyan, Alkyl, Aryl, Acyl, eine heterocyclische Gruppe, Alkoxycarbonyl, Aroxycarbonyl, Sulfonyl, Imino, Imido, Sulfamoyl, Carbonamido oder Sulfonamido; wobei Z¹ und Z² zusammen mit dem C-Atom, an das sie gebunden sind, ein carbocyclisches oder heterocyclisches Ringsystem bilden können.

4. Aufzeichnungsmaterial nach Anspruch 3, dadurch gekennzeichnet, daß es in mindestens einer seiner kupplerhaltigen Schichten eine Verbindung einer der Formeln II und III enthält: 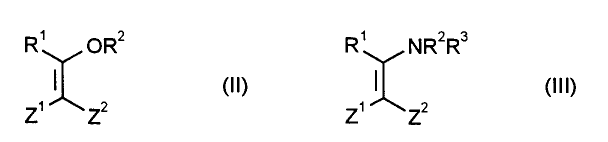 worin bedeuten:
R¹ H, Alkyl, Aryl, -OR² oder -NR²R³;
R² Alkyl, Aryl oder (für -OR² auch): H;
R³ einen Rest wie R² oder gegebenenfalls einen Rest, der zusammen mit R² und dem N-Atom einen heterocyclischen Ring bildet;
Z¹ und Z² unabhängig voneinander: H, Halogen, Cyan, Alkyl, Aryl, Acyl, eine heterocyclische Gruppe, Alkoxycarbonyl, Aroxycarbonyl, Sulfonyl, Imino, Imido, Sulfamoyl, Carbonamido oder Sulfonamido, wobei Z¹ und Z² zusammen mit dem C-Atom, an das sie gebunden sind, ein carbocyclisches oder heterocyclisches Ringsystem bilden können.

5. Verfahren zur Verminderung des Gehaltes an einer Farbentwicklerverbindung in einem der Entsorgung oder der Wiederaufbereitung zuzuführenden gebrauchten fotografischen Behandlungsbad oder dem Überlauf eines solchen gebrauchten fotografischen Behandlungsbades, dadurch gekennzeichnet, daß man dem Behandlungsbad oder dem Überlauf eine Verbindung der Formel I zusetzt: 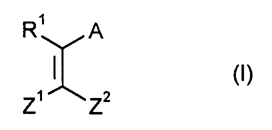 worin bedeuten
A -OR², -SR² oder -NR²R³;
R¹ H, Alkyl, Aryl, Acyl, Sulfonyl oder einen Rest wie A oder einen Rest, der zusammen mit A oder zusammen mit Z¹ einen Ring vervollständigt;
R² H, Alkyl, Aryl, Acyl, Sulfonyl;
R³ einen Rest wie R² oder gegebenenfalls einen Rest, der zusammen mit R² und dem N-Atom einen heterocyclischen Ring bildet,
Z¹ und Z² unabhängig voneinander: H, Halogen, Cyan, Alkyl, Aryl, Acyl, eine heterocyclische Gruppe, Alkoxycarbonyl, Aroxycarbonyl, Sulfonyl, Imino, Imido, Sulfamoyl, Carbonamido oder Sulfonamido; wobei Z¹ und Z² zusammen mit dem C-Atom, an das sie gebunden sind, ein carbocyclisches oder heterocyclisches Ringsystem bilden können.
